# EUROPEAN PATENT APPLICATION

(11) **EP 3 741 312 A1**
(43) Date of publication of application: **25.11.2020**
(21) Application number: 19175841.6
(22) Date of filing: 22.05.2019
(51) Int. Cl.: A61B 17/12, A61B 17/00

(54) **OCCLUDER WITH STRETCHABLE WAIST**

(71) Applicant: Occlutech Holding AG, 8200 Schaffhausen (CH)
(72) Inventor: ISILKI, Cansel, Maslak Mahallesi Sariyer, Istanbul (TR); SOYUTEMIZ, Secil, Bahaelieur, Istanbul (TR); CAVUSOGLU, Olcay, Bayrampasa, Istanbul (TR); MORGAN, Gareth, Denver, Colorado 80220 (US)
(74) Representative: KIPA AB

(57) **Abstract**

The invention discloses an occluder for a structural heart defect, comprising a tubular interlaced structure having a pitch that varies along an axial length of the occluder; a proximal element; a distal element; and an intermediate element extending between the proximal element and the distal element. According to one aspect, at least one transition section of the pitch, from a first pitch to a second pitch, is located closer to the intermediate element of the occluder than an outer circumference of at least one of the proximal elements and the distal element when the occluder is in a relaxed state. According to another aspect, the occluder is extendible from the relaxed state to an axially extended state wherein the load to extension ratio, measured in N/mm, provided by the occluder is less than about 0.75 when extended at least 2 mm.

## Description

### Field of the Invention

The present invention pertains to the field of occluders, such as PDA (Patent Ductus Arteriosus) occluders, PLD (Paravalvular Leak Device), a TAVI (transcatheter aortic valve implantation) PLD, VSD (Ventricular Septal Defect) occluders, LAA (Left Atrial Appendage) occluder, and vascular plugs. In one aspect, the invention comprises an occluder with a tubular interlaced structure having a pitch that varies along an axial length of the occluder, and where at least one transition section of the pitch is located closer to a center axis or an intermediate member of said occluder than an outer circumference a proximal element and/or a distal element when the occluder is in a relaxed state. In another aspect, the invention comprises an occluder that is extendible from the relaxed state to an axially extended state, which is longer in the longitudinal direction of the occluder than the relaxed state, and wherein the load to extension ratio, measured in N/mm, provided by the occluder is less than about 0.75 when extended at least 2 mm. The first aspect and the second aspect can form mutually exclusive embodiments or be combined into a single embodiment.

### Background of the Invention

Occluders provide for full occlusion and allow for minimally invasive procedures for treatment of a variety of structural heart diseases. Interventional techniques for treatment of structural heart disease using occluders have become established routine.

Occluders for treatment of structural heart diseases may have a disc at one end of the device, or two discs, one at each end of opposing ends of a waist. The disc(s) may have enlarged diameters relative a waist. Normally, the disc(s) is/are used as the occluding part of the occluder.

An occluder having two discs can extend between two sides of a heart structure, such as a septum wall, a heart valve, or between the pulmonary artery and the aorta. The discs can be positioned on each side of the heart structure. A waist can extend through the heart structure. Such heart structures can have great variability. To facilitate the large variability of, e.g., length and shape of anatomies, occluders with a single disc have commonly been used for treating some diseases. For single disc occluders, either the disc or the waist can be used as the occluding part. However, the anatomy may distort the occluding waist such that the occluding efficacy of the device is deteriorated. It may be challenging to seat the occluder in the anatomy.

In case an occluder with two discs, designed for a certain clinical/anatomical length, is used in a situation actually requiring an extended/longer length of the waist to suit the anatomy, the discs may be distorted due to the stiffness of the waist. This could deteriorate the occluding efficacy provided by the discs. Other structural heart diseases are treated with occluders that have a uniform diameter, or only a single disc.

Certain anatomies are not suitable for occlusion with any of the previously described occluders.

Therefore, there is a need for an occluder with increased flexibility for treatment of anatomies having a larger variability, such as length and/or shape.

### Summary of the Invention

Accordingly, embodiments of the present invention preferably seek to mitigate, alleviate or eliminate one or more deficiencies, disadvantages or issues in the art, such as the above-identified, singly or in any combination by providing occluder according to the appended patent claims.

The invention is defined by the appended claims.

Embodiments of the invention provide an occluder comprising at least one wire; a tubular interlaced structure made of at least one wire and having a pitch, wherein the pitch varies along an axial length of the occluder; a proximal element having an outer circumference and an inner diameter in a relaxed state of the occluder; a distal element having an outer circumference and an inner diameter in the relaxed state of the occluder; and an intermediate element extending between the proximal element and the distal element.

According to a first aspect, at least one transition section of the pitch, from a first pitch to a second pitch, is located closer to the intermediate element or the central longitudinal axis of the occluder than the outer circumference of at least one of the proximal element and the distal element when the occluder is in the relaxed state.

According to a second aspect, the occluder is extendible in its longitudinal direction, from the relaxed state to an axially extended or elongated state, which is longer in the longitudinal direction of the occluder than the relaxed state. A load to extension ratio, measured in N/mm, provided by the occluder is less than about 0.75 when extended at least 2 mm.

In various embodiments, the first aspect and the second aspect may be mutually exclusive embodiments or combined into a single embodiment. Each of the first aspect and second aspect, separately or together, may be combined with the following embodiments.

In an embodiment, at least one transition section of the pitch may be located between the outer circumference and the inner diameter of at least one of the proximal element and the distal element when the occluder is in the relaxed state.

In an embodiment, at least one of the proximal element and the distal element comprises at least the first pitch, and the intermediate element comprises the second pitch only along an axial length of the intermediate element.

In an embodiment, at least one of the outer circumferences of the proximal element and the distal element is substantially constant when the inner diameter is reduced upon stretching the occluder in the longitudinal direction.

In an embodiment, at least one of the proximal element and the distal element comprises the first pitch only.

In an embodiment, the intermediate element comprises the first pitch and the second pitch along an axial length of the intermediate element.

In an embodiment, the occluder is extendible from the relaxed state to an axially extended state, which is longer in the longitudinal direction of the occluder than the relaxed state.

In embodiments, the load to extension ratio, measured in N/mm, provided by the occluder is less than 0.5 N/mm, preferably less than 0.75 N/mm, even more preferably less than 0.25 N/mm, when extended from at least 2 mm and up to about 6 mm.

In embodiments, a reduction of a cross-sectional diameter of the intermediate element is less than 60%, preferably less than 40%, when the occluder is extended from the relaxed state to the extended state.

In embodiments, the outer circumference of at least one of the proximal element and the distal element is substantially constant when the occluder is extended in length more than 50% of the relaxed state and up to about 150% of the relaxed state.

In embodiments, the intermediate element is at least partly straight or waisted in shape, such as cylindrical or conical, bell-shaped, or hour-glass shaped, relative a longitudinal axis of the occluder when the occluder is in the relaxed state.

In embodiments, the occluder is a PDA occluder, a PLD, a TAVI PLD, a VSD occluder, an LAA (Left Atrial Appendage) occluder, or a VP (vascular plug).

Embodiments of the invention provide an occluder that can be used for occluding anatomies having a wide variety of shapes, and for which occlusion by surgery hitherto may have been the only option available. Particularly, the stretchability of the present invention provides for an occluder that may adapt to the shape of a large variety of anatomies and is therefore very flexible. For example, when used with proximal and/or distal elements of enlarged diameter relative a waist in between, the occluder may provide occlusion with the proximal and distal elements provided at respective ends of an intermediate element.

The term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of, will be apparent and elucidated from the following description of embodiments of the present invention, reference being made to the accompanying drawings, in which
Fig. 1 is a side view of an embodiment of a basic shape of a tubular interlaced structure before heat setting:
Fig. 2a is a side view and cross sectional views taken at the centre of an embodiment of the occluder, across the longitudinal direction and viewed towards the proximal and distal ends, respectively, when the occluder is in the relaxed state;
Fig. 2b is a perspective view of the occluder of Fig. 2a in a relaxed state;
Fig. 3a is a side view and cross sectional views taken at the centre of the occluder of Fig. 2a, across the longitudinal direction and viewed towards the proximal and distal ends, respectively, when the occluder is in an axially extended or elongated state;
Fig. 3b is a perspective view of the occluder of Fig. 2a in the axially extended or elongated state;
Fig. 4a is a side view and cross sectional views taken at the centre of an embodiment of the occluder, across the longitudinal direction and viewed towards the proximal and distal ends, respectively, when the occluder is in the relaxed state;
Fig. 4b is a perspective view of the occluder of Fig. 4a in a relaxed state;
Fig. 5a is a side view and cross sectional views taken at the centre of the occluder of Fig. 4a, across the longitudinal direction and viewed towards the proximal and distal ends, respectively, when the occluder is in an axially extended or elongated state; and
Fig. 5b is a perspective view of the occluder of Fig. 4a in the axially extended or elongated state.

### Description of embodiments

Specific embodiments of the invention now will be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The present description of the current invention is given with reference to an occluder 1, which is exemplified in the embodiment of a PDA (Patent Ductus Arteriosus) occluder. It should be born in mind, however, that the present invention is not limited strictly to the illustrated embodiments, but includes additional embodiments that form other types of occluders, such as a PLD, a TAVI PLD, a VSD occluder, an LAA occluder or a Vascular Plug.

The invention will be described with regard to a first aspect and a second aspect and embodiments thereof. The first aspect and the second aspect may form mutually exclusive embodiments. However, in some embodiments, the first aspect and the second aspect are combined into a single embodiment, which may be further defined by the detailed embodiments described herein. Structures that are common to the first aspect and the second aspect will first be described.

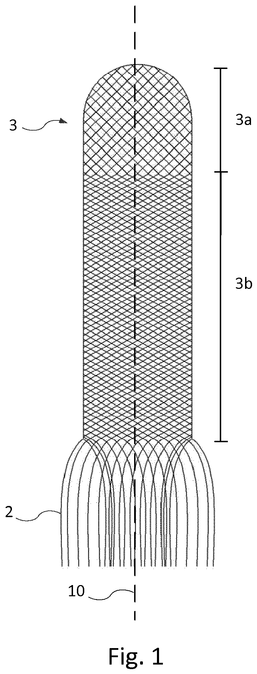

As illustrated in Fig. 1, the occluder 1, comprises at least one wire 2 that may be formed into a tubular interlaced structure 3 (Fig. 1) made of the at least one wire 2. The interlaced structure 3 may also be made of a single wire. The wire 2 may also be referred to as a strand. The wire 2 may be formed by a shape memory material, such as a shape memory metal, for example Nitinol. Particularly, the wire 2 may be formed by a shape memory material with superelastic properties. The wire(s) of the tubular interlaced structure 3 are structured to have pitch, which is the longitudinal distance required for one revolution of the wire around the tubular interlaced structure 3. As can be seen in Fig. 1, the pitch may vary along an axial length of the occluder 1. At a first section 3a, the pitch may be larger than the pitch at a second section 3b. This provides for varying the extensibility or elasticity of the tubular interlaced structure 3 along its length. For example, the pitch of the wires (i.e., the angle defined between the turns of the wires and the axis of the interlaced structure) and the pick of the tubular interlaced structure 3 (i.e., the number of wire crossovers per unit length) may be adjusted as desired for a particular application. For example, the pitch of the wires can be about 90°-130° such as about 100°. Also as an example, the pick count in the second section 3b can be about 7-12 picks/5 mm, such as about 9 picks/5 mm, when the occluder 1 is in the relaxed shape. When the occluder has been stretched about, 4-6 mm, the pitch of the wire can be about 50°-90° and/or the pick count in the second section 3b can be about 12-16 picks/5 mm, such as about 14 picks/5 mm. At the same time, the pitch and/or the pick of the first section 3a can be constant in both the relaxed and stretched shape. Alternatively or additionally, the wires in the first section 3a may be interlaced to form petal like shapes. Hence, the pick of the first section 3a may be substantially constant when the occluder is in the relaxed shape as well as in the stretched state, whereas the second section has a variable pick in the relaxed shape relative the stretched state.

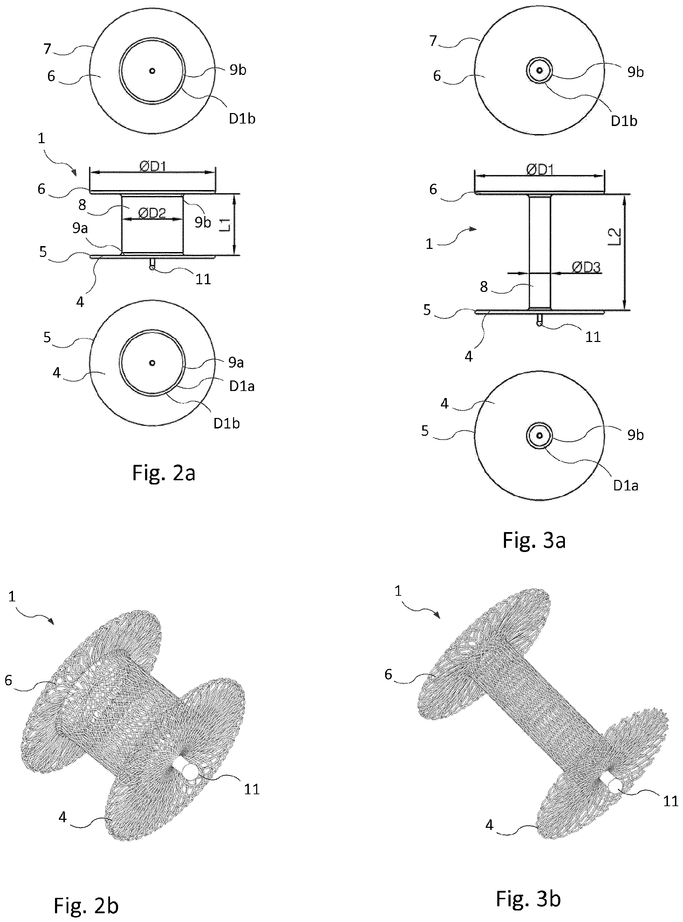

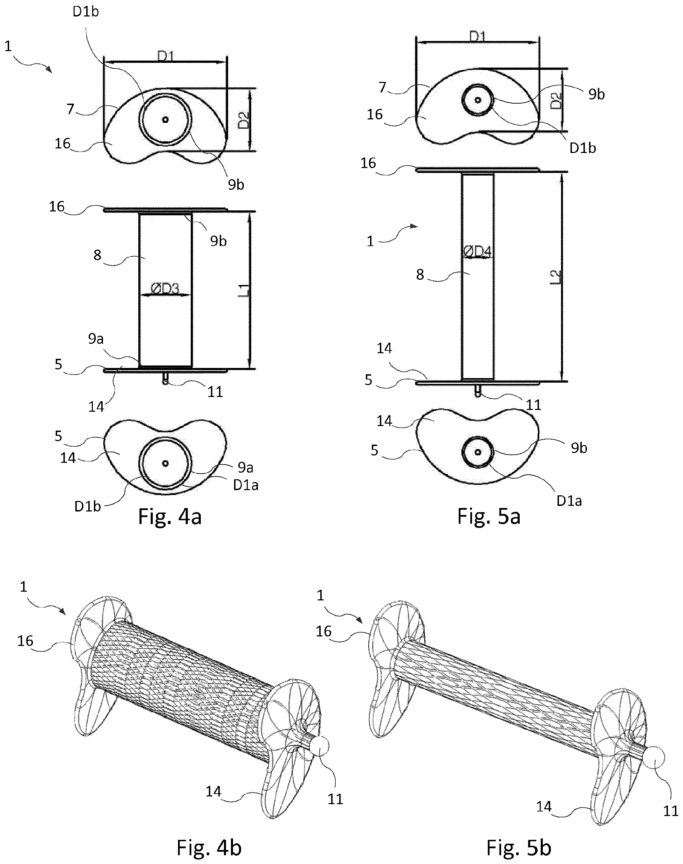

Fig. 1 illustrates the tubular interlaced structure 3 made of a braid of multiple wires in its basic shape before heat treatment and shaping the occluder it its relaxed shape. Moreover, the interlaced structure 3 of Fig. 1 illustrates an embodiment which comprises a first section 3a, and a second section 3b, wherein the first section 3a is distal of the second section 3b. In other embodiments, the interlaced structure 3 comprises a third section, which is formed proximal of the second section 3b. The third section may have a pitch that is different from the pitch of the second section 3b, such as larger than the pitch of the second section 3b. In other embodiments, the pitch of the third section is smaller than the second section 3b. In some embodiments, the pitch of the first section 3a and the third section is the same, which provides for applying substantially equal forces to the second section 3b when the occluder 1 is stretched or elongated as will be described below. In other embodiments, the first section 3a, and optionally the third section, may be formed by wires that are petal shaped, such as is illustrated in Figs. 4b, 5b. Such a shape may also be utilized for the embodiment illustrated in Figs. 2b, 3b.

The interlaced structure 3 of Fig. 1 forms a basic shape that may be heat treated in one or multiple steps to form the shape of the occluder in the relaxed state or non-elongated state and obtain the characteristics of the occluder as will be further described below. Heat treatment of shape memory materials, such as Nitinol, is known as such and will not be further described herein.

Figs. 2a-2b illustrate a relaxed or non-elongated state of the occluder. The occluder 1 may comprise a proximal element 4 having an outer circumference 5 and an inner diameter D1a in the relaxed state of the occluder 1. Additionally or alternatively, the occluder 1 may comprise a distal element 6 having an outer circumference 7 and an inner diameter D1b in the relaxed state of the occluder 1. An intermediate element 8 may extend between the proximal element 4 and the distal element 6.

Each of the proximal element 4 and the distal element 6 may form a disc of the occluder 1. The occluder 1 may comprise the proximal element 4 and/or the distal element 5, i.e. comprise a proximal disc and/or a distal disc. The proximal/distal element 4, 6 may e.g. be substantially circular, oval, substantially square, kidney shaped (e.g. for a TAVI PLD) etc. Furthermore, the proximal/distal element may be cupped, i.e. the outer circumference thereof may be curved in the proximal and distal direction of the occluder 1. In some embodiments, the proximal/distal element 4, 6 is curved from its center. In other embodiments, only a portion of the proximal/distal element 4, 6 is curved. The intermediate member 8 may form a waist of the occluder 1. The maximum outer diameter of the intermediate member 8 may be smaller than the maximum outer diameter of the proximal element 4 and the distal element 6.

In some embodiment, the intermediate element 8 comprises the first pitch and the second pitch along an axial length of the intermediate element 8. Furthermore, the intermediate section 8 may have a varying pitch along its length, such one or several sections with larger and smaller pitch along its length, as can be seen in Figs. 2b and 3b, respectively. This further enhances elastic property of the intermediate section 8. The intermediate section 8 has a substantially higher elasticity in the longitudinal direction of the occluder than the proximal element and the distal element 6.

According to the first aspect, at least one transition section 9a, 9b of the pitch from a first pitch to a second pitch is located closer to the inner diameter D1a and/or the center axis 10 of the occluder than the outer circumference 5, 7 of at least one of the proximal element 4 and the distal element 6 when the occluder 1 is in the relaxed state or non-elongated state. This contributes to the higher elasticity of the intermediate member 8 than at least one of the proximal element 4 and the distal element 6.

According to the second aspect, the occluder 1 may be extended or elongated from the relaxed state to the axially extended state, which is longer in the longitudinal direction of the occluder, than the relaxed state. The occluder 1 may have a load to extension ratio, measured in N/mm. The load to extension ration may be measure as is described below. The load to extension ratio provided by the occluder 1 may be less than about 0.75 N/mm when the occluder is extended or elongated at least 2 mm in the axial direction along the central or longitudinal direction 10 of the occluder 1. This load to extension ratio may be provided without substantially distorting the proximal element 4 and/or the distal element 4. Hence, the ratio defines the higher elasticity of the intermediate member 8 than at least one of the proximal element 4 and the distal element 6.

In the following, embodiments will be described that may be combined with the first aspect only, the second aspect only, or the first aspect and the second aspect in combination.

In some embodiments the load to extension ratio provided by the occluder 1 is less than 0.5 N/mm, preferably less than 0.75 N/mm. In some embodiments, it may even be less than 0.25 N/mm. The load to extension ration may be provided when the occluder 1 is extended or elongated a predefined length in its longitudinal direction. The predefined length may e.g. be within at least 2 mm and up to about 6 mm.

Fig. 2a is a side view the occluder 1 (center view), and cross sectional views taken across the intermediate section 8 and viewed towards the proximal element 4 (top view) and towards the distal element 6 (bottom view). Fig. 3a includes similar views taken of the occluder 1 in the extended state. Each of the proximal element 4 and the distal element 6 may be formed by a double layer of the interlaced structure 3. Hence, the transition section 9a of the proximal element 4 may be located on a distal side of the proximal element 4, as is illustrated at the bottom view of Fig. 2a. Similarly, the transition section of 9b of the distal element 6 may be located on a proximal side of the distal element 6, as is illustrated at the top view of Fig. 2a.

In some embodiment, the at least one transition section 9a, 9b of the pitch is located between the outer circumference 5, 7 and the inner diameter D1a, D1b of at least one of the proximal element 4 and the distal element 6 when the occluder 1 is in the relaxed state. For example, at least one of the proximal element 4 and the distal element 5 may comprise at least the first pitch. The intermediate element 8 may comprise the second pitch only along an axial length of the intermediate element 8. Hence, the intermediate element 8 may be formed of only the second section 3b of the tubular interlaced structure 3. Similarly, the proximal element 4 and/or the distal element 5 may be formed of only the first section 3a/third section of the interlaced structure 3. Hence, the transition section 9a, 9b may bridge the intermediate element 8 and the proximal/distal element 4, 6. Thus, in some embodiments, at least one of the proximal element 4 and the distal element 6 comprises the first pitch only.

Figs. 3a-3b illustrate an extended or elongated state of the occluder 1, wherein the axial length of the occluder 1 is extended or elongated. In this state, the intermediate section 8 of the occluder is extended/elongated. However, the axial length or thickness in the longitudinal direction of the proximal element 4 and/or the distal element 6 remains substantially constant. Furthermore, the inner diameter D3a of the intermediate element 8 in the elongated state of the occluder 1 is reduced relative the relaxed state, whereas the outer circumference of the proximal element 4 and/or the distal element 6 remains substantially constant. This is due to the varying stiffness of the intermediate element 8 and the proximal element 4 and/or the distal element 6.

As is illustrated in Figs 2a-3b, at least one of the outer circumference 5, 7 of the proximal element 4 and the distal element 6, respectively, is substantially constant when the inner diameter D1a, D1b is reduced upon stretching or elongating the occluder 1 along its longitudinal or central axis 10. This may e.g. be provided by providing the varying pitch of the first section 3a, the second section 3b, and optionally of the third section of the tubular interlaced structure 3.

In some embodiments, a cross-sectional diameter of the intermediate element 8 when the occluder is extended in axial length, from the relaxed state to the extended state, is reduced by less than 60%, preferably less than 40%, from diameter D2 to diameter D3.

Similarly, in some embodiments, the outer circumference or diameter 7 of at least one of the proximal element 4 and the distal element 6 is substantially constant when the occluder 1 is extended in length, from a non-extended or relaxed length L1, by more than 50% of the relaxed state and up to about 150% of the relaxed state to an extended or elongated length L2.

In the embodiments illustrated in Figs. 2a-2b, the intermediate element 8 is at least partly straight or waisted in shape, such as cylindrical or conical. However, in other embodiments, the intermediate element 8 is bell-shaped or hour-glass shaped relative the longitudinal axis or center axis 10 of the occluder 1 when the occluder 1 is in the relaxed state. The shape of the intermediate section 8, as well as the shape of the proximal element 8 and/or the distal element 6, may be shaped by one or several molds during heat treatment in one or multiple steps and using the same or different temperatures during the heat treatment of the shape memory material. This is to contribute to the larger elasticity of the intermediate element 8 compared to the proximal element 4 and the distal element 6.

At least one of the ends of the occluder 1 may comprise an attachment element 11 for attaching the occluder 1 to a delivery device for catheter based delivery. The attachment element 11 may be a weld, such as a laser weld. In the illustrated embodiments, the interlaced structure 3 is sack shaped, i.e. the wire(s) 2 start at the proximal end, is interlaced to the distal end, and then returns by interlacing to the proximal end. In other embodiments, the interlaced structure 3 is tubular with an attachment element, such as a clamp, that bundles free ends of the wires 2 at each end. Hence, in such embodiments, one end of the wire 2 is located at the distal end of the occluder and the other end of the wire 2 is located at the proximal end of the occluder 1.

Figs. 4a-4b, 5a-5b illustrates an embodiment wherein with an alternative shape of the outer circumference of the proximal element 14 and the distal element 16. The intermediate element 8 has the same shape as described with regard to Figs. 2a-3b. Furthermore, the proximal element 14 and the distal element 16 and the transition section 9a, 9b to the intermediate element 8 may be designed as has been described with regard to Figs. 2a-3b. Hence, elements having the same configuration as the configuration as described with regard to Figs. 2a-3b have the same reference numerals.

In still other embodiments, the occluder has a single proximal element, such as for an LAA occluder. The intermediate element may form a lobe, such as a cylindrical body having an end portion formed by the interlaced structure and with a diameter not exceeding the diameter of the intermediate element. The intermediate element or lobe may be stretchable as has been described with regard to Figs. 2a-3b. Hooks may be attached to the intermediate element and may engage an anatomical structure, such as an LAA. After the hooks engage the vessel wall, intermediate structure may be stretched and the proximal element positioned outside of the anatomical structure. Due to the stretchability of the intermediate element /lobe, the occluder is more flexible and conforms the shape of the anatomical structure, where in the performance of the occluder is enhanced.

The load to extension ratio may be determined using a tensile testing machine, such as the tensile testing machine with device number 10017 and Brand Lloyd Instruments, by AMETEK Sensors, Test & Calibration. The test procedure comprises:
- attach one end of the occluder 1 to a fixed reference and the other end of the occluder 1 to a tensile testing machine.
- extend the waist of the occluder using the tensile testing machine up to 3N with a tensile test speed of 600 mm/min. 3N is chosen as the maximum applied force to demonstrate stretchability of the occluder at low forces.
- Record the amount of extension or elongation of the occluder without the shape of the proximal and/or the distal element being distorted.
- Release the tension applied to the occluder, and observe the reversibility of the stretch behavior, i.e. that the device can return to its original shape without deformation after release of tension.

The below table illustrates test embodiments A and B falling within the above mentioned ranges of the D1, D2, D3, L1 and L2:

| Test sample | D1 (mm) | D2 (mm) | D3 (mm) | L1 (mm) | L2 (mm) |
|---|---|---|---|---|---|
| Embodiment A | 10 | 4 | 2 | 6 | 10 |
| Embodiment B | 10 | 4 | 2 | 8 | 14 |

The test method was applied to test embodiments A and B defined above and using an mVSD Occluder, size 4, article no. 71VSD04, as reference. The overall shape of embodiments A and B corresponds to the shape illustrated in Figs. 2b and 3b. Hence, embodiments A and B have a proximal element and a distal element shaped as a disc, and an intermediate element in the form of a waist. The disc shape is circular. The mVSD Occluder has an overall similar shape, but has a more uniform and conventional stretchability, i.e. the elasticity of the intermediate element is similar to the stretchability of the proximal element/distal element, and is made from a single pitch braid. The diameter of the intermediate element or waist is 4mm, the diameter of the proximal element and the distal element (discs) is 10 mm, and the height of the waist or intermediate element is 7 mm.

The stretching behavior of the embodiments of the present invention relative the mVSD device is illustrated in the below table:

| Test sample | Initial length of occluder (mm), L1 | Stretched length of occluder (mm), L2 | Force at stretched length (N) | Reversible elongation (mm) | Elongation (%) |
|---|---|---|---|---|---|
| Embodiment A | 6 | 13.6 | 3 | 7.6 | 127 |
| Embodiment B | 8 | 19.3 | 3 | 11.3 | 141 |
| mVSD Occluder | 7 | 9.2 | 3 | 2.2 | 31 |

The test samples had the same raw material and sterilization method. The pitch of the braid and the heat setting varied between embodiments A and B relative the mVSD Occluder. There are numerous combinations of braid patterns and heat settings that may be applied to achieve the above characteristics, which falls within the disclosure as described above with regard to embodiments of the invention.

As can be seen from the above table, the embodiments of the present invention can be elongated or extended substantially longer than the conventional device. Particularly, the test demonstrates that the occluder according to embodiments of the invention may be stretched or elongated reversible, i.e. without distorting the original shape, such as length or disc diameter, of the device after elongation;
- 100% to 150% when the applied load is 3 N
- 50% to 100% when the applied load is 1 N

This should be compared to the regular occluders, exemplified by the mVSD occluder, characterized by being stretched or elongated reversible without distorting the original shape, such as length or disc diameter, of the device after elongation;
- 0% to 35% when the applied load is 3 N
- 0% to 30% when the applied load is 1 N

It should also be appreciated that features disclosed in the foregoing description, and/or in the foregoing drawings and/or following claims both separately and in any combination thereof, be material for realizing the present invention in diverse forms thereof. When used in the following claims, the terms "comprise", "include", "have" and their conjugates mean, "including but not limited to".

The present invention has been described above with reference to specific embodiments. However, other embodiments than the above described are equally possible within the scope of the invention. Different method steps than those described above may be provided within the scope of the invention. The different features and steps of the invention may be combined in other combinations than those described. The scope of the invention is only limited by the appended patent claims.

## Claims

1. Occluder, comprising
at least one wire;
a tubular interlaced structure made of said at least one wire and having a pitch, said pitch varies along an axial length of the occluder;
a proximal element having an outer circumference and an inner diameter in a relaxed state of the occluder;
a distal element having an outer circumference and an inner diameter in the relaxed state of the occluder;
an intermediate element extending between the proximal element and the distal element, wherein
- at least one transition section of said pitch from a first pitch to a second pitch is located closer to the intermediate element or central axis of said occluder than the outer circumference of at least one of the proximal elements and the distal element when the occluder is in the relaxed state.

2. The occluder according to claim 1, wherein said at least one transition section of said pitch is located between the outer circumference and the inner diameter of at least one of the proximal element and the distal element when the occluder is in the relaxed state.

3. The occluder according to claim 1 or 2, wherein at least one of the proximal element and the distal element comprises at least the first pitch, and the intermediate element comprises the second pitch only along an axial length of the intermediate element.

4. The occluder according to any of the previous claims, wherein at least one of said outer circumference of said proximal element and said distal element is substantially constant when said inner diameter is reduced upon stretching said occluder.

5. The occluder according to claim 1 or 2, wherein at least one of the proximal element and the distal element comprises the first pitch only.

6. The occluder according to claim 1, 2 or 5, wherein the intermediate element comprises the first pitch and the second pitch along an axial length of the intermediate element.

7. The occluder according to any of the previous claims, wherein the occluder is extendible from the relaxed state to an axially extended state, which is longer in the longitudinal direction of the occluder, than the relaxed state, and wherein the load to extension ratio, measured in N/mm, provided by the occluder is less than about 0.75 when extended at least 2 mm.

8. The occluder according to claim 7, wherein the load to extension ratio, measured in N/mm, provided by the occluder is less than 0.5 N/mm, preferably less than 0.75 N/mm, even more preferably less than 0.25 N/mm, when extended from at least 2 mm and up to about 6 mm.

9. The occluder according to claim 7 or 8, wherein a reduction of a cross-sectional diameter of the intermediate element is less than 60%, preferably less than 40% when extended from the relaxed state to an extended state.

10. The occluder according to any of the previous claims, wherein the outer circumference of at least one of the proximal element and the distal element is substantially constant when the occluder is extended in length more than 50% of the relaxed state and up to about 150% of the relaxed state .

11. The occluder according to any of the previous claims, wherein the intermediate element is at least partly straight or waisted in shape relative a longitudinal axis of the occluder when the occluder is in the relaxed state.

12. The occluder according to any of the previous claims, wherein the occluder is a PDA occluder, a PLD occluder, a TAVI PLD occluder, a VSD occluder, a mVSD, an LAA occluder, or a vascular plug.

13. Occluder, comprising
at least one wire;
a tubular interlaced structure made of said at least one wire and having a pitch, said pitch varies along an axial length of the occluder;
a proximal element having an outer circumference and an inner diameter in a relaxed state of the occluder;
a distal element having an outer circumference and an inner diameter in the relaxed state of the occluder;
an intermediate element extending between the proximal element and the distal element, wherein
- the occluder is extendible from the relaxed state to an axially extended state, which is longer in the longitudinal direction of the occluder, than the relaxed state, and wherein the load to extension ratio, measured in N/mm, provided by the occluder is less than about 0.75 when extended at least 2 mm.

14. The occluder according to claim 13, at least one transition section of said pitch from a first pitch to a second pitch is located closer to a center axis or the intermediate element of said occluder than the outer circumference of at least one of the proximal element and the distal element when the occluder is in the relaxed state.

15. The occluder according to claim 14, wherein said at least one transition section of said pitch is located between the outer circumference and the inner diameter of at least one of the proximal element and the distal element when the occluder is in the relaxed state.

16. The occluder according to any of claims 13-15, wherein the load to extension ratio, measured in N/mm, provided by the occluder is less than 0.5 N/mm, preferably less than 0.75 N/mm, even more preferably less than 0.25 N/mm, when extended from at least 2 mm and up to about 6 mm.

17. The occluder according to claim any of claims 13-16, wherein a reduction of a cross-sectional diameter of the intermediate element when extended from the relaxed state to an extended state is less than 60%, preferably less than 40%.

18. The occluder according to any of claims 13-17, wherein the outer circumference of at least one of the proximal element and the distal element is substantially constant when the occluder is extended in length more than 50% of the relaxed state and up to about 150% of the relaxed state.

19. The occluder according to any of the previous claims, wherein the occluder is a PDA occluder, a PLD occluder, a TAVI PLD occluder, a VSD occluder, a mVSD, an LAA occluder, or a VP.
